# EUROPEAN PATENT APPLICATION

(11) **EP 4 474 394 A1**
(43) Date of publication of application: **11.12.2024**
(21) Application number: 22924804.2
(22) Date of filing: 03.02.2022
(51) Int. Cl.: C07K 16/18, A61K 39/395, A61P 1/02, C07K 16/46, C12N 15/13, C12P 21/08

(54) **USAG-1 MOLECULE-TARGETING NEUTRALIZING ANTIBODY FOR TOOTH REGENERATION TREATMENT**

(71) Applicant: Kyoto University, Kyoto-shi, Kyoto 606-8501 (JP); University of Fukui, Fukui-shi, Fukui 910-8507 (JP); Aichi Prefecture, Aichi 460-8501 (JP); OSAKA UNIVERSITY, Suita-shi Osaka 565-0871 (JP)
(72) Inventor: TAKAHASHI, Katsu, Kyoto-shi, Kyoto 606-8501 (JP); SUGAI, Manabu, Yoshida-gun, Fukui 910-1193 (JP); TOKITA, Yoshihito, Kasugai-shi, Aichi 480-0392 (JP); TAKAGI, Junichi, Suita-shi, Osaka 565-0871 (JP); MIHARA, Emiko, Suita-shi, Osaka 565-0871 (JP)
(74) Representative: Hoffmann Eitle
(86) International application number: PCT/JP2022/004300
(87) International publication number: WO 2023/148894

(57) **Abstract**

Provided are: an antibody which specifically binds to and neutralizes USAG-1 or an antigen-binding fragment thereof; and a pharmaceutical composition containing the antibody or the antigen-binding fragment.

## Description

### Technical Field

The present invention relates to a neutralizing antibody targeting USAG-1 for the treatment of tooth agenesis or tooth regeneration.

### Background Art

In the majority of patients, acquired diseases such as dental caries and periodontal disease result in tooth agenesis (patients with loss of teeth). As high as 1% incidence rate of congenital tooth agenesis is also reported. Currently, the only treatment method for missing teeth is prosthetic treatment which includes dental implants and dentures, and there is no fundamental treatment method. Numerous studies of tooth regeneration using tissue engineering approaches have been reported. Various cells such as stem cells (Non-Patent Literature 1) are used as cell sources. In addition, in order to allow teeth made in vitro to function in the oral cavity, an "organ primordium method" (Non-Patent Literature 2), a cell manipulation technology for regenerating a dental organ primordium (the rudiment of the organ) in a collagen gel, has been reported. However, owing to cost and safety problems for securing cell sources, tissue engineering approaches have not reached clinical application.

A large number of causative genes for congenital tooth agenesis have been identified, and many of them are common to both human and mouse. For example, RUNX2, MSX1, EDA, WNT10A, PAX9, AXIN2 etc. are known. Among the listed genes, WNT10A gene has been reported to cause congenital tooth agenesis in the largest number of patients. EDA gene is a causative gene for anhidrotic ectodermal dysplasia, which is a representative disease of syndromic congenital tooth agenesis. Congenital tooth agenesis is caused by tooth development stopped prematurely due to defect in the causative gene and suppression of the function of the causative gene.

### Citation List

### Patent Literature

Patent Literature 1: Ohazama, J Denr Res, 2004
Patent Literature 2: Nakao, Nat Methods, 2007

### Summary of Invention

### Problem to be solved by Invention

From a therapeutic viewpoint, the inventors conceived of a novel treatment method to treat congenital tooth agenesis. The new approach promotes differentiation induction from the state of tooth development that has been stopped prematurely to form a complete tooth. Object of the present invention is to provide a technique for treating tooth agenesis which comprises utilizing the differentiation induction inherent in a tooth organ, instead of utilizing surgical tissue transplantation.

### Solution for Problem

As a result of diligent research, the present inventors succeeded in developing a neutralizing antibody targeting USAG-1. Furthermore, they found that administration of the antibody regenerated missing teeth in congenital tooth agenesis model mice and formed supernumerary teeth in congenital tooth agenesis model mice or wild-type mice. Thus the present invention was completed.

That is, the present invention relates to:
[1] An antibody or antigen-binding fragment thereof that specifically binds to and neutralizes USAG-1, comprising three heavy chain complementarity determining regions that comprise amino acid sequences having at least 90% sequence identity with amino acid sequences set forth in SEQ ID NO: 5, SEQ ID NO: 6 and SEQ ID NO: 7 respectively, and/or three light chain complementarity determining regions that comprise amino acid sequences having at least 90% sequence identity with amino acid sequences set forth in SEQ ID NO: 8, SEQ ID NO: 9 and SEQ ID NO: 10 respectively;
[2] The antibody or antigen fragment thereof according to [1], which specifically binds to USAG-1 and neutralizes BMP signaling inhibitory activity of USAG-1;
[3] The antibody or antigen fragment thereof according to [1] or [2], which specifically binds to USAG-1 and neutralizes WNT signaling inhibitory activity of USAG-1;
[4] The antibody or antigen-binding fragment thereof according to any one of [1] to [3], which comprises a heavy chain variable region that comprises an amino acid sequence having at least 90% sequence identity with an amino acid sequence set forth in SEQ ID NO: 3, and/or a light chain variable region that comprises an amino acid sequence having at least 90% sequence identity with an amino acid sequence set forth in SEQ ID NO: 4;
[5] An antibody or antigen-binding fragment thereof that competes with the antibody or antigen-binding fragment thereof according to any one of [1] to [4] for binding to USAG-1;
[6] The antibody or antigen-binding fragment thereof according to any one of [1] to [5], wherein the antibody is a humanized antibody or a chimeric antibody; and
[7] A pharmaceutical composition for dental regenerative therapy, which comprises the antibody or antigen-binding fragment thereof according to any one of [1] to [6].

### Effects of the Invention

In the present invention, we succeeded in regenerating teeth in vivo by using an antibody preparation. Treatment with the antibody preparation of the present invention can be clinically applied as a tooth regenerative therapy in a general dental and oral surgical approach such as conventional tooth extraction, orthodontics, and tooth transplantation.

### Brief Description of Drawings

[FIG. 1] Figure 1 shows USAG-1KO mice established using CRISPR-CAS9.
[FIG. 2] Figure 2 shows experimental results of binding of mouse anti-USAG-1 antibodies to mouse/human USAG-1 proteins.
[FIG. 3] Figure 3 shows sequences of heavy chain and light chain variable regions of antibody.
[FIG. 4] Figure 4 shows the neutralizing activity of the antibody of the present invention on the WNT signaling inhibitory activity and the BMP signaling inhibitory activity of mouse USAG-1.
[FIG. 5] Figure 5 shows results of pull-down assay, showing the interaction between a complex of the mouse anti-USAG-1 antibody with mouse USAG-1 protein and an LRP6-E1E2 domain.

### Mode for carrying out the Invention

USAG-1 (Uterine Sensitization Associated Gene-1) is a bone morphogenetic protein (BMP) antagonist and a Wnt antagonist, and is also called Sostdc-1, Ectodin, or Wise. It is known that in USAG-1 deficient model mice, an increase in BMP signaling is observed, leading to the formation of supernumerary teeth. The present inventors crossed a Runx2-deficient mouse, which is a model mouse for congenital tooth agenesis, with a USAG-1 gene-deficient mouse, which is a model mouse for supernumerary teeth (teeth exceeding the normal number of teeth), to produce a double-knockout mouse. As a result of analysis of the double-knockout mouse, it was found that tooth formation was recovered. Thus it was suggested that inhibition of USAG-1 could treat tooth agenesis.

This time, the present inventors crossed congenital tooth agenesis model mice lacking causative genes Msx1, Eda and Wnt10a other than Runx2 with an USAG-1 gene-deficient mouse that was newly created by CRISPER-CAS9 system as a model mouse for supernumerary teeth, to produce double-knockout mice. As a result of analysis of the double-knockout mice, it was found that tooth formation was recovered in all the tooth agenesis model mice. Thus it was shown that the treatment by inhibition of USAG-1 can be applied to patients with congenital tooth agenesis caused by various gene mutations.

Then, in the present invention, a human USAG-1 recombinant protein whose activity was confirmed was used as an antigen to produce antibodies. Thus antibodies that specifically bind to USAG-1 were obtained. These antibodies were found to increase BMP signaling and/or Wnt signaling.

Therefore, an aspect of the present invention provides an antibody or an antigen-binding fragment thereof that specifically binds to and neutralizes USAG-1, that is, an anti-USAG-1 neutralizing antibody and an antigen-binding fragment thereof. As used herein unless otherwise specified, USAG-1 means mammalian USAG-1. Examples of the mammal include, but not limited to, a human, a dog, a cat, a horse, a mouse, a ferret, a suncus, a pig, and a monkey, and a human is preferable.

As used herein, neutralizing refers to inhibiting the function of USAG-1. The functions of USAG-1 include, for example, BMP signaling inhibitory activity (also referred to as "BMP antagonist activity") and Wnt signaling inhibitory activity (also referred to as "Wnt antagonist activity"). The antibody or antigen-binding fragment thereof of the present disclosure inhibits the BMP signaling inhibitory activity and/or the Wnt signaling inhibitory activity of USAG-1. Therefore, the antibody or antigen-binding fragment thereof of the present disclosure neutralizes either or both of the BMP signaling inhibitory activity of USAG-1 and the Wnt signaling inhibitory activity of USAG-1. For example, the antibody or antigen-binding fragment thereof of the present disclosure includes, but not limited to, an antibody or an antigen-binding fragment thereof that specifically binds to USAG-1 and neutralizes the BMP signaling inhibitory activity of USAG-1 and does not neutralize the Wnt signaling inhibitory activity of USAG-1, and an antibody or an antigen-binding fragment thereof that specifically binds to USAG-1 to neutralize the Wnt signaling inhibitory activity of USAG-1 and does not neutralize the BMP signaling inhibitory activity of USAG-1. As used herein, "inhibition" includes suppression and reduction.

The neutralizing activity of an antibody or an antigen-binding fragment may be determined by a conventional method. The activity of neutralizing the BMP antagonist activity of USAG-1 (also referred to as "BMP antagonist neutralizing activity") can be measured in vitro by, for example, an ALP (alkaline phosphatase) assay or a reporter assay. In the ALP assay, for example, osteoblast progenitor cells and the like are cultured in the presence of BMP with addition of USAG-1 protein and an antibody or an antigen-binding fragment thereof, and ALP generated when differentiation into osteoblasts is induced is measured. The activity of neutralizing the Wnt antagonist activity of USAG-1 (also referred to as "Wnt antagonist neutralizing activity") can be determined in vitro, for example, by a reporter assay. In the reporter assay, for example, a vector containing a promoter region that reacts with BMP or Wnt, ligated to a reporter gene such as luciferase is introduced into a cell, the cell is cultured in the presence of BMP or Wnt with addition of USAG-1 protein and an antibody or an antigen-binding fragment thereof, and expressed luciferase activity is measured. The BMP antagonist activity to be neutralized by the anti-USAG-1 antibody or antigen-binding fragment thereof of the present disclosure may be an antagonist activity against any BMP family. For example, the anti-USAG-1 antibody or antigen-binding fragment thereof of the present disclosure may neutralize the antagonist activity against BMP2, BMP4, BMP6, BMP7, etc. The Wnt antagonist activity to be neutralized by the anti-USAG-1 antibody or antigen-binding fragment thereof of the present disclosure may be an antagonist activity against any Wnt family. For example, the anti-USAG-1 antibody or antigen-binding fragment thereof of the present disclosure may neutralize the antagonist activity against Wnt-1, Wnt-3, etc.

Furthermore, in the present invention, one of the obtained antibodies was sequenced and analyzed. Then, the variable regions and complementarity determining regions of the antibody were determined. The antibody comprises a heavy chain comprising an amino acid sequence set forth in SEQ ID NO: 1 and a light chain comprising an amino acid sequence set forth in SEQ ID NO: 2, and the heavy chain comprises a heavy chain variable region (SEQ ID NO: 3) comprising heavy chain complementarity determining regions comprising amino acid sequences set forth in SEQ ID NO: 5, SEQ ID NO: 6 and SEQ ID NO: 7, and the light chain comprises a light chain variable regions (SEQ ID NO: 4) comprising light chain complementarity determining regions set forth in SEQ ID NO: 8, SEQ ID NO: 9 and SEQ ID NO: 10. The antibody particularly has Wnt antagonist neutralizing activity.

Therefore, in an aspect of the present invention, the above-described antibody and a mutant thereof are provided as the antibody or antigen-binding fragment thereof of the present disclosure. An example of the antibody or a mutant thereof includes an antibody or an antigen-binding fragment thereof that specifically binds to and neutralizes USAG-1 and comprises three heavy chain complementarity determining regions comprising amino acid sequences having at least 80%, 85%, 90%, 91%, 92%, 93%, 94%, 95%, 96%, 97%, 98% or 99% sequence identity with amino acid sequences set forth in SEQ ID NO: 5, SEQ ID NO: 6 and SEQ ID NO: 7 respectively, or three light chain complementarity determining regions comprising amino acid sequences having at least 80%, 85%, 90%, 91%, 92%, 93%, 94%, 95%, 96%, 97%, 98% or 99% sequence identity with amino acid sequences set forth in SEQ ID NO: 8, SEQ ID NO: 9 and SEQ ID NO: 10 respectively. Preferably provided is an antibody or an antigen-binding fragment thereof that specifically binds to and neutralizes USAG-1 and comprises three heavy chain complementarity determining regions comprising amino acid sequences set forth in SEQ ID NO: 5, SEQ ID NO: 6 and SEQ ID NO: 7 respectively, or three light chain complementarity determining regions comprising amino acid sequences set forth in SEQ ID NO: 8, SEQ ID NO: 9 and SEQ ID NO: 10 respectively. More preferably provided is an antibody or an antigen-binding fragment thereof that specifically binds to and neutralizes USAG-1 and comprises three heavy chain complementarity determining regions consisting of amino acid sequences having at least 80%, 85%, 90%, 91%, 92%, 93%, 94%, 95%, 96%, 97%, 98% or 99% sequence identity with amino acid sequences set forth in SEQ ID NO: 5, SEQ ID NO: 6 and SEQ ID NO: 7 respectively, or three light chain complementarity determining regions consisting of amino acid sequences having at least 80%, 85%, 90%, 91%, 92%, 93%, 94%, 95%, 96%, 97%, 98% or 99% sequence identity with amino acid sequences set forth in SEQ ID NO: 8, SEQ ID NO: 9 and SEQ ID NO: 10 respectively. Still more preferably provides is an antibody or an antigen-binding fragment thereof that specifically binds to and neutralizes USAG-1 and comprises three heavy chain complementarity determining regions consisting of amino acid sequences set forth in SEQ ID NO: 5, SEQ ID NO: 6 and SEQ ID NO: 7 respectively, or three light chain complementarity determining regions consisting of amino acid sequences set forth in SEQ ID NO: 8, SEQ ID NO: 9 and SEQ ID NO: 10 respectively.

A further example of the antibody or a mutant thereof includes an antibody or an antigen-binding fragment thereof that specifically binds to and neutralizes USAG-1, comprises three heavy chain complementarity determining regions comprising amino acid sequences set forth in SEQ ID NO: 5, SEQ ID NO: 6 and SEQ ID NO: 7 respectively, or three light chain complementarity determining regions comprising amino acid sequences set forth in SEQ ID NO: 8, SEQ ID NO: 9 and SEQ ID NO: 10 respectively, and comprises substitution, deletion, insertion or addition of one to several amino acid residues in at least one of the above-mentioned amino acid sequences. Preferably provided is an antibody or an antigen-binding fragment thereof that specifically binds to and neutralizes USAG-1, comprises three heavy chain complementarity determining regions consisting of amino acid sequences set forth in SEQ ID NO: 5, SEQ ID NO: 6 and SEQ ID NO: 7 respectively, or three light chain complementarity determining regions consisting of amino acid sequences set forth in SEQ ID NO: 8, SEQ ID NO: 9 and SEQ ID NO: 10 respectively, and comprises substitution, deletion, insertion or addition of one to several amino acid residues in at least one of the above-mentioned amino acid sequences.

A further example of the antibody or a mutant includes an antibody or an antigen-binding fragment thereof that specifically binds to and neutralizes USAG-1 and comprises a heavy chain variable region comprising an amino acid sequence having at least 80%, 85%, 90%, 91%, 92%, 93%, 94%, 95%, 96%, 97%, 98% or 99% sequence identity with an amino acid sequence set forth in SEQ ID NO: 3 or a light chain variable region comprising an amino acid sequence having at least 80%, 85%, 90%, 91%, 92%, 93%, 94%, 95%, 96%, 97%, 98% or 99% sequence identity with an amino acid sequence set forth in SEQ ID NO: 4. Preferably provided is an antibody or an antigen-binding fragment thereof that specifically binds to and neutralizes USAG-1 and comprises a heavy chain variable region comprising an amino acid sequence set forth in SEQ ID NO: 3 or a light chain variable region comprising an amino acid sequence set forth in SEQ ID NO: 4. More preferably provided is an antibody or an antigen-binding fragment thereof that specifically binds to and neutralizes USAG-1 and comprises a heavy chain variable region consisting of an amino acid sequence having at least 80%, 85%, 90%, 91%, 92%, 93%, 94%, 95%, 96%, 97%, 98% or 99% sequence identity with an amino acid sequence set forth in SEQ ID NO: 3 or a light chain variable region consisting of an amino acid sequence having at least 80%, 85%, 90%, 91%, 92%, 93%, 94%, 95%, 96%, 97%, 98% or 99% sequence identity with an amino acid sequence set forth in SEQ ID NO: 4. Still more preferably provided is an antibody or an antigen-binding fragment thereof that specifically binds to and neutralizes USAG-1 and comprises a heavy chain variable region consisting of an amino acid sequence set forth in SEQ ID NO: 3 or a light chain variable region consisting of an amino acid sequence set forth in SEQ ID NO: 4.

A further example of the antibody or a mutant thereof includes an antibody or an antigen-binding fragment thereof that specifically binds to and neutralizes USAG-1, comprises a heavy chain variable region comprising an amino acid sequence set forth in SEQ ID NO: 3 or a light chain variable region comprising an amino acid sequence set forth in SEQ ID NO: 4, and comprises substitution, deletion, insertion or addition of one to several amino acid residues in at least one of the above-mentioned amino acid sequences. Preferably provided is an antibody or an antigen-binding fragment thereof that specifically binds to and neutralizes USAG-1, comprises a heavy chain variable region consisting of an amino acid sequence set forth in SEQ ID NO: 3 or a light chain variable region consisting of an amino acid sequence set forth in SEQ ID NO: 4, and comprises substitution, deletion, insertion or addition of one to several amino acid residues in at least one of the above-mentioned amino acid sequences.

A further example of the antibody or a mutant thereof includes an antibody or an antigen-binding fragment thereof that specifically binds to and neutralizes USAG-1, and comprises three heavy chain complementarity determining regions comprising amino acid sequences having at least 80%, 85%, 90%, 91%, 92%, 93%, 94%, 95%, 96%, 97%, 98% or 99% sequence identity with amino acid sequences set forth in SEQ ID NO: 5, SEQ ID NO: 6 and SEQ ID NO: 7 respectively and three light chain complementarity determining regions comprising amino acid sequences having at least 80%, 85%, 90%, 91%, 92%, 93%, 94%, 95%, 96%, 97%, 98% or 99% sequence identity with amino acid sequences set forth in SEQ ID NO: 8, SEQ ID NO: 9 and SEQ ID NO: 10 respectively. Preferably provided is an antibody or an antigen-binding fragment thereof that specifically binds to and neutralizes USAG-1, and comprises three heavy chain complementarity determining regions comprising amino acid sequences set forth in SEQ ID NO: 5, SEQ ID NO: 6 and SEQ ID NO: 7 respectively and three light chain complementarity determining regions comprising amino acid sequences set forth in SEQ ID NO: 8, SEQ ID NO: 9 and SEQ ID NO: 10 respectively. More preferably provided is an antibody or an antigen-binding fragment thereof that specifically binds to and neutralizes USAG-1, and comprises three heavy chain complementarity determining regions consisting of amino acid sequences having at least 80%, 85%, 90%, 91%, 92%, 93%, 94%, 95%, 96%, 97%, 98% or 99% sequence identity with amino acid sequences set forth in SEQ ID NO: 5, SEQ ID NO: 6 and SEQ ID NO: 7 respectively and three light chain complementarity determining regions consisting of amino acid sequences having at least 80%, 85%, 90%, 91%, 92%, 93%, 94%, 95%, 96%, 97%, 98% or 99% sequence identity with amino acid sequences set forth in SEQ ID NO: 8, SEQ ID NO: 9 and SEQ ID NO: 10 respectively. Still more preferably provides is an antibody or an antigen-binding fragment thereof that specifically binds to and neutralizes USAG-1, and comprises three heavy chain complementarity determining regions consisting of amino acid sequences set forth in SEQ ID NO: 5, SEQ ID NO: 6 and SEQ ID NO: 7 respectively and three light chain complementarity determining regions consisting of amino acid sequences set forth in SEQ ID NO: 8, SEQ ID NO: 9 and SEQ ID NO: 10 respectively.

A further example of the antibody or a mutant thereof includes an antibody or an antigen-binding fragment thereof that specifically binds to and neutralizes USAG-1, comprises three heavy chain complementarity determining regions comprising amino acid sequences set forth in SEQ ID NO: 5, SEQ ID NO: 6 and SEQ ID NO: 7 respectively and three light chain complementarity determining regions comprising amino acid sequences set forth in SEQ ID NO: 8, SEQ ID NO: 9 and SEQ ID NO: 10 respectively, and comprises substitution, deletion, insertion or addition of one to several amino acid residues in at least one of the above-mentioned amino acid sequences. Preferably provided is an antibody or an antigen-binding fragment thereof that specifically binds to and neutralizes USAG-1, comprises three heavy chain complementarity determining regions consisting of amino acid sequences set forth in SEQ ID NO: 5, SEQ ID NO: 6 and SEQ ID NO: 7 respectively and three light chain complementarity determining regions consisting of amino acid sequences set forth in SEQ ID NO: 8, SEQ ID NO: 9 and SEQ ID NO: 10 respectively, and comprises substitution, deletion, insertion or addition of one to several amino acid residues in at least one of the above-mentioned amino acid sequences.

A further example of the antibody or a mutant thereof includes an antibody or an antigen-binding fragment thereof that specifically binds to and neutralizes USAG-1, and comprises a heavy chain variable region comprising an amino acid sequence having at least 80%, 85%, 90%, 91%, 92%, 93%, 94%, 95%, 96%, 97%, 98% or 99% sequence identity with an amino acid sequence set forth in SEQ ID NO: 3 and a light chain variable region comprising an amino acid sequence having at least 80%, 85%, 90%, 91%, 92%, 93%, 94%, 95%, 96%, 97%, 98% or 99% sequence identity with an amino acid sequence set forth in SEQ ID NO: 4. Preferably provided is an antibody or an antigen-binding fragment thereof that specifically binds to and neutralizes USAG-1, and comprises a heavy chain variable region comprising an amino acid sequence set forth in SEQ ID NO: 3 and a light chain variable region comprising an amino acid sequence set forth in SEQ ID NO: 4. More preferably provided is an antibody or an antigen-binding fragment thereof that specifically binds to and neutralizes USAG-1, and comprises a heavy chain variable region consisting of an amino acid sequence having at least 80%, 85%, 90%, 91%, 92%, 93%, 94%, 95%, 96%, 97%, 98% or 99% sequence identity with an amino acid sequence set forth in SEQ ID NO: 3 and a light chain variable region consisting of an amino acid sequence having at least 80%, 85%, 90%, 91%, 92%, 93%, 94%, 95%, 96%, 97%, 98% or 99% sequence identity with an amino acid sequence set forth in SEQ ID NO: 4. Still more preferably provided is an antibody or an antigen-binding fragment thereof that specifically binds to and neutralizes USAG-1, and comprises a heavy chain variable region consisting of an amino acid sequence set forth in SEQ ID NO: 3 and a light chain variable region consisting of an amino acid sequence set forth in SEQ ID NO: 4.

A further example of the antibody or a mutant thereof includes an antibody or an antigen-binding fragment thereof that specifically binds to and neutralizes USAG-1, comprises a heavy chain variable region comprising an amino acid sequence set forth in SEQ ID NO: 3 and a light chain variable region comprising an amino acid sequence set forth in SEQ ID NO: 4, and comprises substitution, deletion, insertion or addition of one to several amino acid residues in at least one of the above-mentioned amino acid sequences. Preferably provided is an antibody or an antigen-binding fragment thereof that specifically binds to and neutralizes USAG-1, comprises a heavy chain variable region consisting of an amino acid sequence set forth in SEQ ID NO: 3 and a light chain variable region consisting of an amino acid sequence set forth in SEQ ID NO: 4, and comprises substitution, deletion, insertion or addition of one to several amino acid residues in at least one of the above-mentioned amino acid sequences.

As used herein, the term "several" means about 2 to 10, and preferably means, depending on the length of an amino acid sequence, about 2 to 7, for example, 3, 4, 5, or 6. As used herein, the "substitution" may be a conservative or non-conservative substitution, and preferably a conservative substitution. Conservative substitution is known to those skilled in the art, and refers to a substitution that does not affect the biological activity of the resulting molecule. Examples of conservative amino acid substitution include a substitution of alanine to glycine or serine, a substitution of arginine to lysine or histidine, a substitution of asparagine to glutamine or histidine, a substitution of aspartic acid to glutamic acid or asparagine, a substitution of cysteine to serine or alanine, a substitution of glutamine to asparagine, a substitution of glutamic acid to aspartic acid or glutamine, a substitution of glycine to alanine, a substitution of histidine to asparagine or glutamine, a substitution of isoleucine to leucine or valine, a substitution of leucine to isoleucine or valine, a substitution of lysine to arginine or histidine, a substitution of methionine to leucine, isoleucine or tyrosine, a substitution of phenylalanine to tyrosine, methionine or leucine, a substitution of proline to alanine, a substitution of serine to threonine, a substitution of threonine to serine, a substitution of tryptophan to tyrosine or phenylalanine, a substitution of tyrosine to tryptophan or phenylalanine, and a substitution of valine to isoleucine or leucine.

As used herein, the sequence identity may be determined in optimal alignment of two sequences according to a conventional method. For example, the sequence identity may be determined using an algorithm known in the art, such as BLAST or FASTA. The antibody or antigen-binding fragment thereof of the present disclosure may comprise substitution, deletion, insertion or addition of an amino acid residue(s) within the above-mentioned range of sequence identity.

Further, in another aspect of the present invention, an antibody or an antigen-binding fragment thereof that binds to a whole extent or a part of the same epitope as an epitope on USAG-1 to which the antibody or a mutant thereof, or an antigen-binding fragment thereof binds is provided as the antibody or antigen-binding fragment thereof of the present disclosure. Furthermore, the present invention provides an antibody or an antigen-binding fragment thereof that competes with the antibody or a mutant thereof, or an antigen-binding fragment thereof for binding to USAG-1 or for binding to a whole extent or a part of an epitope on USAG-1.

As used herein, the term "competition" means that an antibody or an antigen-binding fragment thereof competes with a reference antibody (e.g., the above-mentioned antibody or a mutant thereof, or an antigen-binding fragment thereof) in a binding assay using an USAG-1 protein or polypeptide. For example, if a test antibody or an antigen-binding fragment thereof reduces the binding of a reference antibody to an USAG-1 protein or polypeptide in the binding assay, the test antibody "competes" with the reference antibody. An antibody that competes with a reference antibody, for example, reduces the binding of the reference antibody to an antigen protein or polypeptide by at least about 40%, preferably at least about 50%, more preferably at least about 60%, still more preferably at least about 80%, or still more preferably at least 90%. The competitive binding assay can be performed by known methods in the art including, but not limited to, ELISA, flow cytometry, SPR (surface plasmon resonance), and BLI (Bio-Layer Interferometry).

Epitope binning is a technique for classifying two or more antibodies based on their epitopes, for example, by using SPR or BLI. In epitope binning, for example, an antigen protein (target) is added to a biosensor on which a reference antibody is immobilized to allow the reference antibody to bind the target, the biosensor holding a complex of the reference antibody and the target is reacted with a test antibody, and then, binding of the test antibody to the biosensor (i.e., binding of the test antibody to the target captured by the reference antibody immobilized on the biosensor) and dissociation of the binding are analyzed. If the test antibody shares the same epitope with the reference antibody, the test antibody cannot bind to the biosensor because the epitope on the target is already occupied by the binding of the reference antibody. Conversely, if the test antibody recognizes a different epitope from that recognized by the reference antibody, the test antibody can bind to the biosensor. Furthermore, if the test antibody recognizes a region sterically close to an epitope recognized by the reference antibody, the test antibody cannot bind to the biosensor because the binding of the reference antibody to the target interferes with the binding of the test antibody to the epitope. Thus, use of epitope binning enables to determine whether two or more antibody clones compete for binding to a target protein.

The antibody or antigen-binding fragment thereof of the present disclosure binds to USAG-1 or a whole extent or a part of an epitope on USAG-1 at a KD of for example 1 µM or less, preferably 100 nM or less, more preferably 50 nM or less, still more preferably 30 nM or less, still more preferably 10 nM or less, still more preferably 8 nM or less, or still more preferably 5 nM or less.

In the present invention, the antibody is preferably an isolated antibody. In the present invention, the antibody may be a polyclonal antibody or a monoclonal antibody. In the present invention, the antibody may be a human antibody, a humanized antibody, a chimeric antibody, or a multispecific antibody (for example, a bispecific antibody). The humanized antibody includes a human immunoglobulin (recipient antibody) in which the complementarity determining regions (CDRs) of a recipient are replaced by residues from the CDRs of a non-human species (donor antibody) having desired specificity, affinity and binding ability. Optionally, the Fv framework region (FR) residues of the human immunoglobulin may be replaced by the corresponding non-human residues. In addition, the humanized antibody may comprise residues that are not found in the recipient antibody or the donor antibody. Generally, humanized antibodies comprise at least one variable region, typically two variable regions, in which all or substantially all CDRs are replaced by non-human immunoglobulin CDRs and all or substantially all of FR regions consist of human immunoglobulin sequences. The chimeric antibody includes an antibody produced by a genetic recombination technique in which variable regions derived from a donor antibody are linked to constant regions of a recipient antibody. The above antibodies can be produced by methods known in the art.

In the present invention, examples of the antigen-binding fragment include, but not limited to, F(ab')₂, Fab', Fab, Fv, rIgG, Fd, a linear antibody, ScFv, Fv-clasp, a minibody, a diabody, a triabody, a tetrabody, a single domain antibody (nanobody), and a multispecific antibody formed from antibody fragments. The antibody fragments can be prepared by methods known in the art.

An isolated nucleic acid encoding the antibody or antigen-binding fragment thereof of the present disclosure is also included in the present invention.

The antibody or antigen-binding fragment thereof of the present disclosure specifically binds to USAG-1 to inhibit the function of USAG-1 and then induce tooth formation. Accordingly, another aspect of the present invention provides a pharmaceutical composition for dental regeneration therapy comprising the antibody or antigen-binding fragment thereof of the present disclosure. The dental regeneration includes, for example, regeneration of a missing tooth (recovery of a missing tooth), and formation of a new tooth such as a third dentition.

The pharmaceutical composition of the present disclosure may contain a pharmaceutically acceptable carrier, and an additive such as a stabilizer or an excipient, in addition to the antibody or antigen-binding fragment thereof of the present disclosure. Examples of the pharmaceutically acceptable carrier include, but not limited to, physiological saline, buffer, glycol, glycerol, gelatin, gelatin hydrogel, polylactic acid, collagen sponge, agarose, polyvinyl alcohol, alginic acid, fibrin gel, an ethylenevinyl acetate copolymer, and a lactic acid-glycolic acid copolymer. Examples of the additive include, but not limited to, a carbohydrate such as glucose, sucrose or dextran, an antioxidant such as ascorbic acid or glutathione, a chelating agent, and a low molecular weight protein. Those skilled in the art can -appropriately select the carrier and additive as mentioned above based on an administration form, administration route or the like of the pharmaceutical composition. Pharmaceutical composition of the present disclosure can be produced using the antibody or antigen-binding fragment thereof, and the additive as appropriate by a conventional method.

Examples of the form of the pharmaceutical composition of the present disclosure include a tablet, a powder, a capsule, a granule, a syrup, a sustained release tablet, a sustained release capsule, an enteric coated drug, an intercalating drug, an infusion, and an injection. A preferable example thereof is an injection. The pharmaceutical composition of the present disclosure is systemically or topically administered. The administration route may be appropriately selected by those skilled in the art, and examples thereof include, but not limited to, oral, nasal, subcutaneous, intravenous, intramuscular, and intraosseous administration. The pharmaceutical composition of the present disclosure may be locally administered, for example, to the site of tooth formation.

In the present invention, the dental regenerative therapy includes the treatment of congenital tooth agenesis and the treatment of acquired tooth loss. Congenital tooth agenesis that can be treated with the pharmaceutical composition of the present disclosure is not particularly limited, and may also include congenital tooth agenesis due to any causative gene. Examples of congenital tooth agenesis that can be treated with the pharmaceutical composition of the present disclosure include, but not limited to, congenital tooth agenesis whose causative gene is RUNX2, MSX1, EDA, WNT10A, PAX9, or AXIN2. Preferable examples thereof include congenital tooth agenesis whose causative gene is RUNX2, MSX1, EDA, or WNT10A. Further, the antibody of the present disclosure induced the formation of supernumerary teeth in wild-type mice. Therefore, the pharmaceutical composition of the present disclosure can induce tooth formation even in normal individuals in which the causative gene of tooth agenesis is not deficient and individuals losing teeth after birth.

Pharmaceutical composition of the present disclosure may be administered to a mammal. Examples of the mammal include a human, a dog, a cat, a horse, a mouse, a ferret, a suncus, a pig, and a monkey. A preferable example thereof is a human.

A dose of the pharmaceutical composition of the present disclosure is not particularly limited. The dose can be appropriately determined by those skilled in the art based on the amount of the antibody or antigen-binding fragment thereof of the present disclosure contained in the pharmaceutical composition, the body weight of a subject to be administered, etc. so that a desired dose of the antibody or antigen-binding fragment thereof of the present disclosure can be administered. For example, the antibody or antigen-binding fragment thereof of the present disclosure is administered in an amount that produces a neutralizing activity such that BMP signaling is increased by at least 30%, preferably at least 60% and/or a neutralizing activity such that Wnt signaling is increased by at least 30%, preferably at least 60%, as compared with the case where the antibody or antigen-binding fragment thereof of the present disclosure is not administered. The neutralizing activities may be determined based on the activity measured in vitro by, for example, an ALP assay or a reporter assay.

A further aspect of the present invention provides a method of regenerating a tooth which comprises administering the antibody or antigen-binding fragment thereof of the present disclosure to a subject in need. The above-mentioned pharmaceutical composition can be used as the antibody or antigen-binding fragment thereof of the present disclosure. The subject in need is a subject having a missing tooth, and examples of the subject include mammals as mentioned above. A route of administration and a dose of the antibody or antigen-binding fragment thereof of the present disclosure, and the treatment for tooth regeneration are as described above for the pharmaceutical composition of the present disclosure.

Hereinafter, the present invention will be described in more detail with reference to Examples which the present invention is not limited to.

### Example 1

### Preparation of antibody

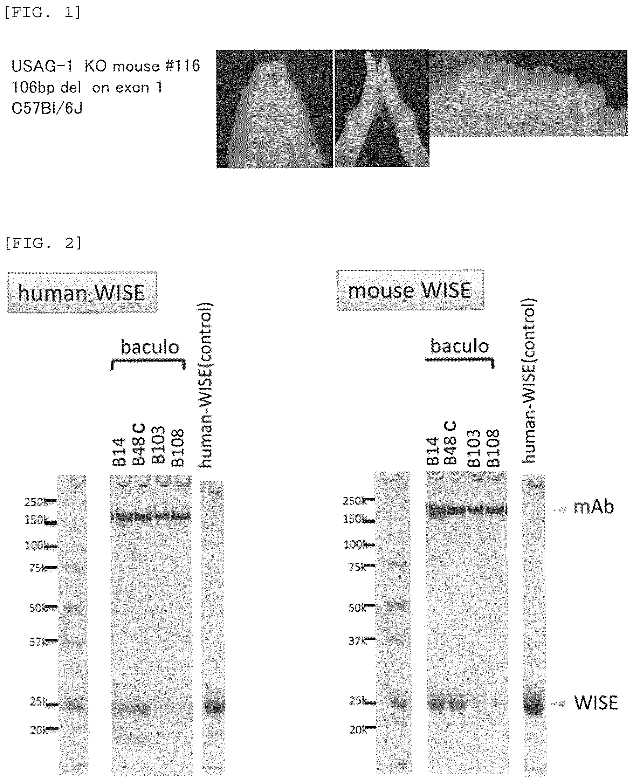

For preparation of mouse USAG-1 neutralizing antibodies, a rat USAG-1 protein derived from a baculovirus expression system was used as an antigen. The neutralizing antibodies were prepared by the iliac lymph node method using USAG-1 KO (#116) mice that was established using CRISPR-CAS9 (FIG. 1). In a Wnt reporter assay using HEK293 cells, the Wnt inhibitory activity of the baculovirus expression system-derived rat USAG-1 protein was confirmed. In an ALP assay with addition of BMP7 using C2C12 cells, the BMP inhibitory activity of the baculovirus expression system-derived rat USAG-1 protein was confirmed. Primary screening of the obtained antibody clones was performed by ELISA using the immunizing antigen (the baculovirus expression system-derived rat USAG-1 protein) and the *Escherichia coli* expression system-derived human USAG-1 protein, and a large number of positive wells were found. Based on a cut-off value of 1.0, 111 clones were selected. After expansion, the clones were subjected to sandwich ELISA using the immunizing antigen. When a cut-off value of absorbance was set to 0.5 or more (His tag) and 1.4 or more (Myc tag), positive wells were found in about half of the clones. As a result of measurement of antibody subclasses, they were found to be IgG1, 2a, 2b, and G3.

The neutralizing activity of each antibody was confirmed as follows. In an experimental system in which an ALP activity in C2C12 cells increased by addition of 300 ng/ml of BMP7 (manufactured by R & D systems) was suppressed by addition of 300 ng/ml of a mammalian cell expression system-derived rat USAG-1 protein (manufactured by MyBiosource), each antibody was added to confirm neutralization of the suppressed ALP activity. The antibodies were classified into a group of those having mild neutralizing activity (*: 60-100% neutralizing activity), a group of those having moderate neutralizing activity (**: 100-140% neutralizing activity), and a group of those having high neutralizing activity (***: 140% or more neutralizing activity). For a BMP reporter assay, a commercially available cell line incorporating BRE-Luc, BMP Responsive Reporter Osteoblast Cell Line (Briter cell) (manufactured by Kerafast, Inc.) was used. In an experimental system in which a luciferase activity expressed by addition of 300 ng/ml of BMP7 was suppressed by addition of 300 ng/ml of the mammalian cell expression system-derived rat USAG-1 protein, each antibody was added to confirm neutralization of the suppressed luciferase activity. The antibodies were classified into a group of those having mild neutralizing activity (*: 40-60% neutralizing activity) and a group of those having moderate neutralizing activity (**: 60% or more neutralizing activity). For the WNT reporter assay, an expression plasmid for expressing a TOP-Flash reporter gene having a DNA sequence to bind transcription factor TCF that activates downstream of the WNT signaling, a WNT1 gene, and a reporter gene under the control of an HSV-thymidine kinase promoter for obtaining an internal standard value was introduced into HEK293 cells, and the cells were cultured with addition of the mammalian cell expression system-derived rat USAG-1 protein at a concentration (EC50) for producing 50% of the maximum inhibitory effect on Wnt signaling. An addition amount of the USAG-1 protein was determined in advance. A culture supernatant of an antibody-producing hybridoma was added to the cells so as to be 25%, 20% or 10% in a medium for screening. The experiment was performed three times for evaluation. When added at 25%, the antibodies were classified into a group of those having mild neutralizing activity (*: a luminescence correction value of 1.5-2.0) and a group of those having moderate neutralizing activity (**: a luminescence correction value of 2.0 or more). When added at 20%, the antibodies were classified into a group of those having mild neutralizing activity (*: a luminescence correction value of 1-1.1) and a group of those having moderate neutralizing activity (**: a luminescence correction value of 1.1 or more). When added at 10%, the antibodies were classified into a group of those having mild neutralizing activity (*: a luminescence correction value of 1-1.1) and a group of those having moderate neutralizing activity (**: a luminescence correction value of 1.1 or more) . The % of neutralizing activity was calculated based on the activity (100%) under the condition of no addition of USAG-1 protein. Neutralizing activity based on the luminescence value was calculated as a value relative to the activity (1) when no antibody was added. Finally, a mouse anti-USAG-1 neutralizing antibody (B48C) was obtained.

Further, a binding assay was performed using moue/human N-terminal PA-tagged USAG-1 proteins to confirm that the obtained mouse anti-USAG-1 neutralizing antibody B48C recognizes the human USAG-1 protein in the same way as three antibodies (B14, B103, B108) obtained by the previous study (WO2021/010346). Each of the mouse anti-USAG-1 neutralizing antibodies [5 µg in 250 µl protein A/G IgG binding buffer (Pierce^{™}) + 250 µl PBS] was captured on Protein A sepharose (30 ul) (room temperature, 1.5 hours). A culture supernatant (0.75 mL) of Expi293F cells transiently expressing a human or mouse N-terminal PA-tagged USAG-1 (WISE) recombinant protein was added to the Protein A sepharose, and then incubated (room temperature, 2 hours). Washing with a PBS buffer was performed 3 times to remove unbound proteins. All proteins bound to the sepharose were eluted, and bands were detected by SDS-PAGE electrophoresis and CBB (Coomassie Brilliant Blue) staining. As a result, all of the tested mouse anti-USAG-1 neutralizing antibodies bound to both the mouse USAG-1 and the human USAG-1 protein (FIG. 2).

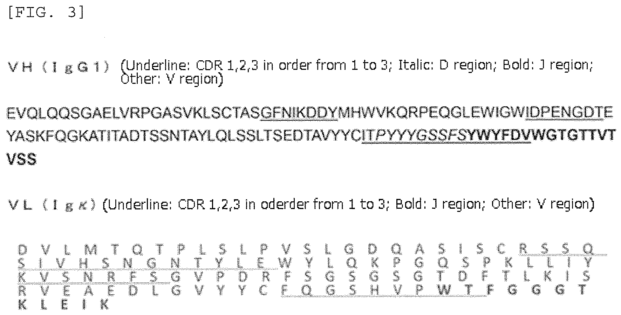

Further, antibody B48C was sequenced. A full-length heavy chain sequence containing a signal sequence and a full-length light chain sequence containing a signal sequence of the antibody are shown in SEQ ID NO: 1 and SEQ ID NO: 2, respectively. Variable regions of the antibody are shown in FIG. 3.

### Example 2

### Epitope binning

The five mouse USAG-1 neutralizing antibodies (E12, E16, E37, E48, E57) that were obtained using the *Escherichia coli* expression system-derived human USAG-1 protein as an antigen in the previous study (WO2021/010346), the three mouse USAG-1 neutralizing antibodies (B14, B103, B108) that were obtained using the baculovirus expression system-derived rat USAG-1 protein as an antigen in the previous study (WO2021/010346), and the neutralizing antibody (B48C) prepared in Example 1 were subjected to epitope binning. Epitope binning was performed using Octet (registered trademark) Red (manufactured by Pall ForteBio). Briefly, each of the nine antibodies was immobilized on a biosensor as a capture antibody, a target comprising a purified full-length recombinant mouse USAG-1 was added to bind the capture antibody, and then the biosensor was reacted with a test antibody to detect a binding signal. This cycle was repeated in succession using 9 test antibodies including the same antibody as used for capture. An antibody that does not compete with the capture antibody immobilized on the biosensor for the recognition site can bind to the captured USAG-1 protein, and thus the signal increases. In contrast, a competing antibody weakly binds to the captured USAG-1 protein, and thus no increase or little increase in the signal was observed. Based on the signal data obtained, the 9 antibodies were grouped based on their epitopes. Results are shown in Table 1.

**[Table 1]**

| Group | Antibody |
|---|---|
| 1 | E12 |
| | E37 |
| 2 | E16 |
| | E48 |
| | E57 |
| | B14 |
| 3 | B48C |
| 4 | B103 |
| | B108 |

### Example 3

### In vitro test of antibody - 1

For the antibody obtained in Example 1, affinity (KD values) was determined by using a BLI method based on Octet (registered trademark) Red (manufactured by Pall ForteBio) . Specifically, each antibody was immobilized on the biosensor, the purified recombinant mouse USAG-1 protein was added at three different concentrations (10 nM, 30 nM, 100 nM), and binding and dissociation curves were obtained. The affinities were calculated by subjecting the obtained curves to global fitting with an analysis program attached to the Octet device. As a result, a KD value of 3.26 nM was shown.

### Example 4

### In vitro test of antibody - 2

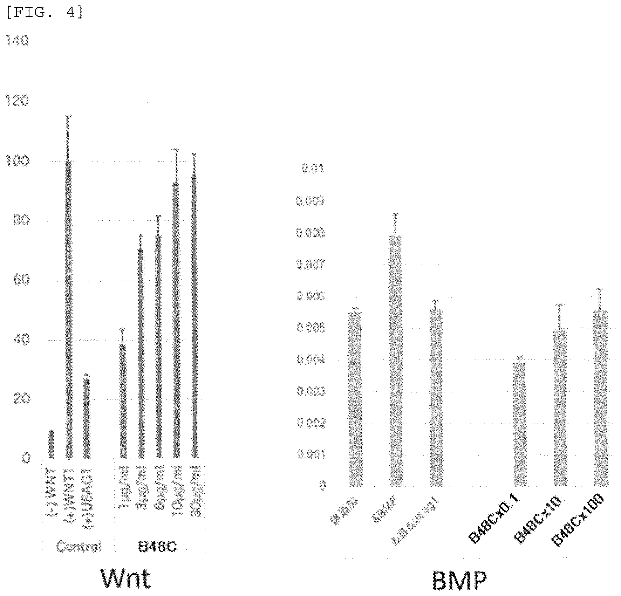

The BMP and Wnt signaling inhibition neutralizing activities of the antibody B48C that was sequenced in Example 1 were determined. Experiments were carried out using mouse USAG-1 recombinant protein. A mouse USAG-1 (WISE) recombinant protein with a PA tag added to the N-terminal was transiently expressed in Expi293F cells, and a stable expression line was established. Affinity purification was performed using a PA tag system to obtain 0.2 mg of PA-mUSAG-1 (WISE) from 150 mL of a culture supernatant. The purified PA-mUSAG-1 (WISE) protein was shown to have a molecular weight of about 28 kDa, which is close to the theoretical value (24 kDa), by electrophoresis under reduction (R) and non-reduction (NR). The N-terminal PA-tagged mouse USAG-1 (WISE) protein derived from the mammalian cell Expi293F cell expression system showed dose-dependent WNT signaling inhibitory activity in the WNT reporter assay and dose-dependent BMP signaling inhibitory activity in the BMP ALP assay. The mouse USAG-1 protein whose activity was confirmed was used to confirm the neutralizing activity of the above-mentioned antibody. In the WNT reporter assay, cells into which a vector containing a luciferase gene ligated to a promoter and a vector for expressing Wnt1 (1 µg) were introduced were cultured in a medium with addition of 1 µg of the mouse USAG-1 recombinant protein and the antibody in an amount of 1, 3, 6, 10 or 30 µg/ml medium, and luciferase activity was measured. In the BMP ALP assay, C2C12 cells were cultured with 30 ng/ml of the mouse USAG-1 recombinant protein and a 0.1-fold (3 ng/ml), 10-fold (300 ng/ml) or 100-fold (3 µg/ml) amount of the antibody in the presence of 30 ng/ml of BMP7, and ALP activity was measured. Results are shown in FIG. 4. In the WNT reporter assay, the antibody was shown to neutralize the WNT signaling inhibitory activity of USAG-1 by almost 100%.

### Example 5

### In vitro test of antibody - 3 (Pull-down assay with Protein A sepharose using PA-tagged USAG-1 and E1E2)

Wnt binds to Frizzled and its co-receptor, a low-density lipoprotein receptor-related protein (LRP) 5/6 receptor to transmit a signal in cells. LRP6 has four extracellular domains (E1 to E4). Among the domains, E1E2 is known to be involved in the binding of USAG-1. Specifically, USAG-1 binds to the E1E2 region of LRP6 to inhibit Wnt signaling. Thus, the mouse anti-USAG-1 neutralizing antibody B48C obtained in Example 1 as well as the neutralizing antibodies (E12, E16, E37, E48, E57, B14, B103, B108) that were obtained by the previous study (WO2021/010346) were used to determine whether the binding of USAG-1 to LRP6-E1E2 was inhibited.

Each mouse anti-USAG-1 neutralizing antibody (5 µg in 1 ml PBS) was captured on Protein A sepharose (30 ul) (room temperature, 1.5 hours). A culture supernatant (1 mL) of Expi293F cells transiently expressing a N-terminal PA-tagged mouse USAG-1 recombinant protein was added to the Protein A sepharose, and then incubated (room temperature, 2 hours). Then, a culture supernatant (1 mL) expressing LRP6-E1E2 (a region of amino acid numbers 1 to 629 of human LRP6, fused to a His tag) was added, and incubated (room temperature, 2.5 hours). Washing with a PBS buffer (1 mL) was performed 3 times to remove unbound proteins. All proteins bound to the sepharose were eluted, and bands were detected by SDS-PAGE electrophoresis and Oriole gel fluorescence staining. Results are shown in FIG. 26. In FIG. 26, the right panel shows results obtained when the same experiment was performed except that the mouse anti-USAG-1 neutralizing antibody was not captured (no mAb) as a negative control, when a complex of the mouse USAG-1 recombinant protein and LRP6-E1E2 was immunoprecipitated with a PA-tagged antibody NZ-1 (WISE + E1E2 By NZ1) as a positive control, and when the expression level of LRP6-E1E2 only was precipitated with a Ni-NTA resin capable of adsorbing the His tag (E1E2 By NiNTA).

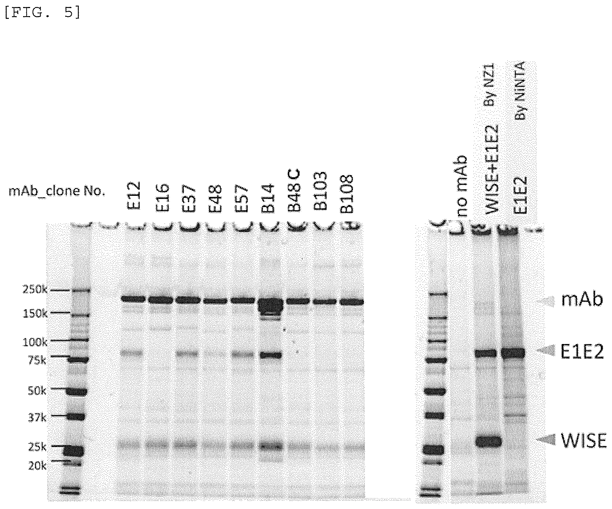

As a result, it was found that LRP6-E1E2 did not bind to a complex of antibody B48C with USAG-1 (FIG. 5). Therefore, it was found that the epitope of the antibody overlaps or is in a region sterically close to the LRP6 binding site of USAG-1, and the antibody binds to USAG-1 to inhibit the binding of USAG-1 to LRP6, and thereby the inhibition of Wnt signaling by USAG-1 is neutralized.

### Example 6

### In vivo administration test of antibody - 1 (Mouse)

The antibody obtained in Example 1 was intraperitoneally administered in a single dose to mother mice pregnant with congenital tooth agenesis model mouse due to EDA homozygous - deficiency, congenital tooth agenesis model mouse due to EDA heterozygous deficiency, or wild-type mice (16 µg/g body weight). In born offspring mice, the presence of supernumerary teeth and fused teeth and the presence or absence of recovery of missing teeth were examined. Results are shown in Table 2. The antibody particularly recovered missing teeth in the EDA-deficient mice. Herein, the term "recovery" means that a born EDA-deficient mouse has a tooth at the site (does not lose M3) where a tooth is normally lost in an EDA-deficient mouse.

**[Table 2]**

| | EDA^{+/+} (wild- type) mouse | EDA^{+/-} mouse | EDA^{-/-} mouse | |
|---|---|---|---|---|
| Antibody | Supernumerary tooth/Fused tooth | Supernumerary tooth/Fused tooth | Recovery | Supernumerary tooth/Fused tooth |
| B48C | 0/1 | 0/1 | 3/4 (75%) | 1/4 |

### Example 7

### In vivo administration test of antibody - 2 (Mouse)

The antibody obtained in Example 1 was intraperitoneally administered in a single dose to mother mice pregnant with congenital tooth agenesis model mouse Wnt10a homozygous deficient mice, congenital tooth agenesis model mouse Wnt10a heterozygous deficient mice, or wild-type mice (16 µg/g body weight). In born offspring mice, the presence of supernumerary teeth and fused teeth and the presence or absence of recovery of missing teeth were examined. Results are shown in Table 3. The antibody induced the formation of supernumerary teeth and fused teeth in the homozygous deficient mice.

**[Table 3]**

| | Wnt10a^{+/+} (wild-type) mouse | Wnt10a^{+/-} mouse | Wnt10a^{-/-} mouse |
|---|---|---|---|
| Antibody | Supernumerary tooth/Fused tooth | Supernumerary tooth/Fused tooth | Supernumerary tooth/Fused tooth |
| B48C | 0/2 | 0/9 | 2/5 (40%) |

### Industrial Applicability

The antibody or antigen-binding fragment thereof of the present disclosure can be used for the treatment of congenital tooth agenesis and acquired tooth loss. In addition, the antibody or antigen-binding fragment thereof of the present disclosure is effective for the formation of the third dentition. Thus, the antibody or antigen-binding fragment thereof of the present disclosure leads to development of a molecular-targeted drug for tooth regeneration in the pharmaceutical field and establishment of a dental regenerative therapy based on formation of the third dentition.

## Claims

1. An antibody or antigen-binding fragment thereof that specifically binds to and neutralizes USAG-1, comprising three heavy chain complementarity determining regions that comprise amino acid sequences having at least 90% sequence identity with amino acid sequences set forth in SEQ ID NO: 5, SEQ ID NO: 6 and SEQ ID NO: 7 respectively, and/or three light chain complementarity determining regions that comprise amino acid sequences having at least 90% sequence identity with amino acid sequences set forth in SEQ ID NO: 8, SEQ ID NO: 9 and SEQ ID NO: 10 respectively.

2. The antibody or antigen fragment thereof according to claim 1, which specifically binds to USAG-1 and neutralizes BMP signaling inhibitory activity of USAG-1.

3. The antibody or antigen fragment thereof according to claim 1 or 2, which specifically binds to USAG-1 and neutralizes WNT signaling inhibitory activity of USAG-1.

4. The antibody or antigen-binding fragment thereof according to any one of claims 1 to 3, which comprises a heavy chain variable region that comprises an amino acid sequence having at least 90% sequence identity with an amino acid sequence set forth in SEQ ID NO: 3, and/or a light chain variable region that comprises an amino acid sequence having at least 90% sequence identity with an amino acid sequence set forth in SEQ ID NO: 4.

5. An antibody or antigen-binding fragment thereof that competes with the antibody or antigen-binding fragment thereof according to any one of claims 1 to 4 for binding to USAG-1.

6. The antibody or antigen-binding fragment thereof according to any one of claims 1 to 4, wherein the antibody is a humanized antibody or a chimeric antibody.

7. A pharmaceutical composition for dental regenerative therapy, which comprises the antibody or antigen-binding fragment thereof according to any one of claims 1 to 6.
